# EUROPEAN PATENT APPLICATION

(11) **EP 2 463 360 A1**
(43) Date of publication of application: **13.06.2012**
(21) Application number: 11192181.3
(22) Date of filing: 06.12.2011
(51) Int. Cl.: C12N 5/00

(54) **Methods for cell culture using a synthetic defined collagen mimetic surface**

(30) Priority: 08.12.2010 US 420860 P; 05.12.2011 US 310988
(71) Applicant: Becton, Dickinson and Company, Franklin Lakes, NJ 07417 (US)
(72) Inventor: Saxena, Deepa, Framingham, MA 01701 (US); Chen, Xiaoxi, Natick, MA 01760 (US); Abraham, Elizabeth J., Andover, MA 01801 (US)
(74) Representative: von Kreisler Selting Werner

(57) **Abstract**

The present invention discloses methods for enhancing cell attachment, cell proliferation and cell function using a surface which mimics a collagen coated surface. Advantageously, such methods employ a xeno-free, synthetic, chemically defined surface.

## Description

### Cross-Reference to Related Application

This application claims the benefit of U.S. Provisional Application No. 61/420,860, filed December 8, 2010, the contents of which are incorporated by reference herein.

### Field of the Invention

The present invention relates to methods for enhancing cell attachment, proliferation and function using a surface which mimics a collagen coated surfaces. More particularly, the present invention relates to methods employing a xeno-free, synthetic surface.

### Background of the Invention

Cell culture models rely on the ability of cells to attach to, proliferate and function in a manner *in vitro* comparable to that *in vivo.* In particular, various culture models employ keratinocytes or hepatocytes to test compounds *in vitro* prior to use *in vivo.* For example, keratinocyte cultures are used for predicting skin irritation and hepatocyte cultures are used for predicting hepatotoxicity. The use of collagen coated cell culture surfaces to support cell attachment and cell function is problematic as the collagen used for coating is generally of human or other animal origin and thus, often poorly defined. Furthermore, the use of such human or other animal derived collagen can be extremely problematic in human therapeutic applications where an immunogenic response that leads to rejection of transplanted cells may result. Although isolated human collagen can be used for coating such surfaces, the cost associated therewith is very high and may still result in an immunogenic response. Additionally, as with recombinant collagen, variability in cell culture may result from different batches of isolated collagen due to variability in the contaminants present therein. Additionally, variability in cell culture may arise from the self-coating process itself which is generally employed for both isolated and recombinant collagen. Thus, there is a need for methods of enhancing cell attachment, proliferation and function using a xeno-free, synthetic, chemically defined surface that mimics collagen.

### Summary of the Invention

The present invention provides methods of using a xeno-free, synthetic, chemically defined surface for cell culture which provides cell attachment and functionality comparable to a collagen coated surface. In particular, cell attachment of keratinocytes, pancreatic cancer cells and hepatocytes is comparable to that of a collagen (*i.e.,* BD BioCoat Collagen1) coated surface. Additionally, keratinocyte cell proliferation is comparable to that of a collagen (*i.e.,* BD BioCoat Collagen1) coated surface. Furthermore, CYP450 basal activity and induction in hepatocytes are comparable to a collagen (*i.e.,* BD BioCoat Collagen1) coated surface. Such methods are particularly desirable as the surface used therein avoids the issues associated with human or other animal-derived collagen which is poorly defined and may also elicit an immune response in therapeutic applications. Likewise, such methods are especially preferred in cell culture assays as the culture conditions are more chemically defined.

In one aspect, the present invention provides methods for cell culture comprising contacting a suspension of cells to a surface wherein at least a portion of the surface comprises a coating thereon of a compound comprising amino acid sequence GPCGPPGPPGPPGPPGPPGFX₁GERGPPGPPGPPGPPGPPGPC (SEQ ID NO: 1) wherein X₁ represents hydroxyproline and incubating the cells under conditions suitable for cell culture; wherein cell attachment to the surface is comparable to cell attachment of the suspension of cells to a collagen coated surface. In one embodiment, the cells are keratinocytes. In another embodiment, the cells are pancreatic cancer cells. In yet another embodiment, the cells are hepatocytes.

In one embodiment, the number of cells attached to the surface is at least 2-fold greater relative to a control surface without any extracellular matrix protein coating thereon. In one embodiment, wherein the cells are keratinocytes, the number of cells attached to the surface is at least 3-fold greater relative to a control surface without any extracellular matrix protein coating thereon. In another embodiment, wherein the cells are pancreatic cancer cells, the number of cells attached to the surface is at least 10-fold greater relative to a control surface without any extracellular matrix protein coating thereon.

In yet another embodiment, the level of basal activity of cytochrome P₄₅₀ 3A4 in hepatocytes attached to the surface is comparable to the level of basal activity of cytochrome P₄₅₀ 3A4 in hepatocytes attached to a collagen coated surface. In still yet another embodiment, cytochrome P₄₅₀ 3A4 induction is comparable to the level of cytochrome P450 induction of hepatocytes attached to a collagen coated surface.

In one embodiment, the surface is a cell culture vessel. In another embodiment, the surface is a microcarrier.

In one embodiment, the cells are incubated at 37°C in a humidified incubator with 5% CO₂. In one embodiment, the cells are incubated for at least 24 hours.

In another aspect, the present invention provides a cell cultured using the methods of the present invention.

These and other features of the invention will be better understood through a study of the following detailed description.

### Brief Description of the Figures

**Figure 1A** is an image of human hepatocyte cells (lot 170) attached to a tissue culture surface that has a coating thereon of collagen (*i.e.,* BD BioCoat Collagen 1).

**Figure 1B** is an image of human hepatocyte cells (lot 170) attached to a tissue culture surface that has a coating thereon of GPCGPPGPPGPPGPPGPPGFX₁GERGPPGPPGPPGPPGPPGPC (SEQ ID NO: 1) wherein X₁ represents hydroxyproline.

**Figure 1C** is an image of human hepatocyte cells (lot 94) attached to a tissue culture surface that has a coating thereon of collagen (*i.e.,* BD BioCoat Collagen 1).

**Figure 1D** is an image of human hepatocyte cells (lot 94) attached to a tissue culture surface that has a coating thereon of GPCGPPGPPGPPGPPGPPGFX₁GERGPPGPPGPPGPPGPPGPC (SEQ ID NO: 1) wherein X₁ represents hydroxyproline.

**Figure 1E** is an image of human hepatocyte cells (lot 197) attached to a tissue culture surface that has a coating thereon of collagen (*i.e.,* BD BioCoat Collagen 1).

**Figure 1F** is an image of human hepatocyte cells (lot 197) attached to a tissue culture surface that has a coating thereon of GPCGPPGPPGPPGPPGPPGFX₁GERGPPGPPGPPGPPGPPGPC (SEQ ID NO: 1) wherein X₁ represents hydroxyproline.

**Figure 1G** is an image of human hepatocyte cells present on a tissue culture surface without any extracellular matrix protein coating thereon.

**Figure 2** is a graph illustrating the basal level of cytochrome P₄₅₀ 3A4 (CYP3A4) induction and the level of enzyme activity represented as pmol/min/mg protein, and fold induction in human hepatocyte cells from 3 different donors attached to a surface that has a coating thereon of GPCGPPGPPGPPGPPGPPGFX₁GERGPPGPPGPPGPPGPPGPC (SEQ ID NO: 1) wherein X₁ represents hydroxyproline (referred to therein as "M") or collagen (*i.e.,* BD BioCoat Collagen 1; referred to therein as "C").

**Figure 3A** is an image of human keratinocyte cells in a defined, animal-free media attached to a tissue culture surface that has a coating of collagen thereon (*i.e.,* BD BioCoat Collagen 1).

**Figure 3B** is an image of human keratinocyte cells in a defined, animal-free media attached to a surface that has a coating thereon of GPCGPPGPPGPPGPPGPPGFX₁GERGPPGPPGPPGPPGPPGPC (SEQ ID NO: 1) wherein X₁ represents hydroxyproline.

**Figure 3C** is an image of human keratinocyte cells in a defined, animal-free media present on a tissue culture surface without any extracellular matrix protein coating thereon.

**Figure 4** is a graph illustrating the level of absorbance at 490nm following MTS assay for quantitation of human keratinocyte present on a surface that has a coating thereon of GPCGPPGPPGPPGPPGPPGFX₁GERGPPGPPGPPGPPGPPGPC (SEQ ID NO: 1) wherein X₁ represents hydroxyproline (referred to therein as "Mimetic") or collagen (*i.e.,* BD BioCoat Collagen1; referred to therein as "BD BioCoat Col1").

**Figure 5A** is an image of human keratinocyte cells in a defined media attached to a surface that has a coating thereon of GPCGPPGPPGPPGPPGPPGFX₁GERGPPGPPGPPGPPGPPGPC (SEQ ID NO: 1) wherein X₁ represents hydroxyproline.

**Figure 5B** is an image of human keratinocyte cells in a defined media attached to a tissue culture surface that has a coating thereon of collagen (*i.e.,* BD BioCoat Collagen 1).

Figure 5C is an image of human keratinocyte cells in a defined media present on a tissue culture surface without any extracellular matrix protein coating thereon.

**Figure 6** is a graph illustrating the level of absorbance at 490nm following MTS assay for quantitation of human keratinocyte cells present on a surface with a coating thereon of GPCGPPGPPGPPGPPGPPGFX₁GERGPPGPPGPPGPPGPPGPC (SEQ ID NO: 1) wherein X₁ represents hydroxyproline; (referred to therein as "A"), collagen (*i.e.,* BD BioCoat Collagen 1; referred to therein as "B"), or a tissue culture surface without any extracellular matrix protein coating thereon (referred to therein as "C").

**Figure 7A** is an image of human pancreatic cancer cells present on a surface that has a coating thereon of GPCGPPGPPGPPGPPGPPGFX₁GERGPPGPPGPPGPPGPPGPC (SEQ ID NO: 1) wherein X₁ represents hydroxyproline.

**Figure 7B** is an image of human pancreatic cancer cells present on a tissue culture surface that has a coating of collagen thereon (*i.e.,* BD BioCoat Collagen 1).

**Figure 7C** is an image of human pancreatic cancer cells present on a tissue culture surface without any extracellular matrix protein coating thereon.

**Figure 8** is a graph illustrating the level of absorbance at 490nm following MTS assay for quantitation of human pancreatic cancer cells present on a surface with a coating thereon of GPCGPPGPPGPPGPPGPPGFX₁GERGPPGPPGPPGPPGPPGPC (SEQ ID NO: 1) wherein X₁ represents hydroxyproline (referred to therein as "Mimetic"), collagen *(i.e.,* BD BioCoat Collagen 1; referred to therein as "Coll"), or a tissue culture surface without any extracellular matrix protein coating thereon (referred to therein as "TC").

### Detailed Description of the Invention

As used herein the following terms shall have the definitions set forth below.

As used herein, the terms "mimic" and "mimics" with regard to the comparison of a cell culture surface coated with a compound of the present invention with a cell culture surface coated with collagen refers to the relative similarity in one or more functional characteristics being assessed. Desirably, quantification thereof would reveal at least 90% similarity in at least one functional characteristic.

The compounds used in the present invention for coating surfaces may be produced using conventional recombinant technologies or synthetic techniques (e.g., solid phase synthesis). Similarly, such compounds may be purified using conventional techniques to a degree suitable for a given application. In one embodiment, the compounds have a level of purity that is at least 90%. Advantageously, synthetic synthesis of such compounds can provide a level of purity that is at least 95% or greater. Desirably, such compounds have a level of purity that is 97% or greater. In certain applications, such as therapeutics, it is particularly preferred that the compounds are synthesized.

It is understood that one of skill in the art could substitute one or more amino acids of the amino acid sequence described herein for coating surfaces without compromising the ability of the resultant compound when coated on a surface for cell culture to mimic one or more functional properties of a collagen coated surface.

For example, one or more amino acids of the compound disclosed herein for coating a surface may be conservatively substituted. A conservative substitution being defined as the side chain of the respective amino acid being replaced by a side chain of similar chemical structure and polarity, the side chain being derived from a genetically coded or not genetically coded amino acid. Families of amino acids of this kind having similar side chains are known in the art. They include, for instance, amino acids having basic side chains (lysine, arginine, histidine), acidic side chains (aspartic acid, glutamic acid), uncharged polar side chains (glycine, asparagine, glutamine, serine, threonine, tyrosine, cysteine), non-polar side chains (alanine, valine, leucine, isoleucine, proline, phenylalanine, methionine, tryptophan), beta-branched side chains (threonine, valine, isoleucine) and aromatic side chains (tyrosine, phenylalanine, tryptophane, histidine).

Surfaces of the present invention modified using a compound comprising amino acid sequence GPCGPPGPPGPPGPPGPPGFX₁GERGPPGPPGPPGPPGPPGPC (SEQ ID NO: 1) wherein X₁ represents hydroxyproline are useful for cell culture where one or more functional properties of collagen are desirable.

Surfaces modified with a compound described herein may employ either passive (*i.e.*, non-covalent) coating, covalent immobilization of the compound or any other method of deposition of the compound.

Surfaces modified with a compound described herein for use in cell culture include cell culture vessels and microcarriers. Suitable cell culture vessels for use in the present invention are well known to one of skill in the art. Examples of suitable vessels include, but are not limited to, dishes, flasks, multi-well plates, and microscopic slides. Microcarriers suitable for cell culture are also well known to one of skill in the art. See, *e.g.,* Nie, Biotechnol. Prog., 25(1):20-31 (2009).

Advantageously, cells cultured using the surfaces of the present invention are suitable for therapeutic application (e.g., in wound healing) and avoid problems inherent to the use of isolated collagen from a different source which may otherwise elicit an immunogenic response and even lead to rejection of transplanted cells.

### EXAMPLES

A compound having amino acid sequence GPCGPPGPPGPPGPPGPPGFX₁GERGPPGPPGPPGPPGPPGPC (SEQ ID NO: 1) wherein X₁ represents hydroxyproline was synthesized using a commercially available custom peptide synthesis service. This compound was then added on a surface suitable for cell culture.

To explore the ability of a surface modified by the compound to enhance cell attachment, proliferation and function comparable to a collagen coated surface, cells were seeded and monitored on both such surfaces under the same culture conditions. In brief, human hepatocyte, human keratinocyte, or human pancreatic cancer cells were cultured according to supplier's instructions.

Cryo preserved inducible human hepatocytes from BD Biosciences (BD Biosciences Cat No. 454550) were purified using Heptocyte Purification Kit (BD Biosciences Cat No. 454500) according to the supplier's instructions. In brief, cells were seeded at a density of 3x10⁵ cells/well in a 24-well plate according to the supplier's instructions on BD BioCoat Collagen1 or a surface coated with a compound having amino acid sequence GPCGPPGPPGPPGPPGPPGFX₁GERGPPGPPGPPGPPGPPGPC (SEQ ID NO: 1) wherein X₁ represents hydroxyproline. Cells were incubated in a humidified incubator at 37°C with 5% CO₂. After 2-4 hrs, media was removed and cells were re-fed with 400 µl/well Hepatostim complete media as per supplier's instructions (BD BioSciences Cat No. 355056).

Human (neonatal) epidermal keratinocytes were purchased from Invitrogen (Cat # C-001-5C) and cultured onto a surface coated with a compound having amino acid sequence GPCGPPGPPGPPGPPGPPGFX₁GERGPPGPPGPPGPPGPPGPC (SEQ ID NO: 1) wherein X₁ represents hydroxyproline or animal-derived collagen (*i.e.,* BD BioCoat Collagen1) in Epilife media supplemented with EDGS (containing animal components) or S7 (animal-component-free) supplements, according to the manufacurer's instructions. Briefly cells were seeded at 25,000/cm² and cultured at 37°C in a humidified incubator at 5% CO₂. Cells were visualized under the microscope and images were captured. Attachment on the surfaces was quantified by MTS assay day4-day5 post plating. In brief, media was removed by aspiration and 300 µl complete media containing MTS (Promega cat # G3582) was added to each well of a 24 well plate. Cells were incubated for 1 hr at 37°C in a humidified incubator at 5% CO₂. Following incubation, 0.1 ml media was transferred to BD Falcon™ 96 well plate and absorbance was measured at 490 nm.

PANC-1 (human pancreatic carcinoma cell line) cells from ATCC were cultured in DMEM (Invitrogen Cat No. 11885-084) supplemented with 10% fetal bovine serum at 37°C in a humidified incubator with 5% CO₂.

For seeding, media was removed, cells were washed with PBS, and 3 ml of 0.25% Trypsin-EDTA was added to the cells in T-75 flask. Flasks were examined under the microscope, once cells detached from the surface, 10 ml culture media was added to neutralize Trypsin. Cells were transferred to a 15 ml BD Falcon tube and centrifuged at 200 X g for 10 min. Supernatant was removed and the cell pellet washed once with DMEM (Invitrogen cat # 11885-084) having 200 microgram/ml BSA. Cells were resuspended in DMEM having 200 microgram/ml BSA and seeded at 50,000 cells/cm² in 0.5 ml media per well of a 24 well plate. The culture surface was either BD BioCoat Collagen1 which served as a positive control or a surface modified by a peptide having amino acid sequence GPCGPPGPPGPPGPPGPPGFX₁GERGPPGPPGPPGPPGPPGPC (SEQ ID NO: 1) wherein X₁ represents hydroxyproline. Additionally, BD tissue culture treated surface served as negative control. Cells were incubated at 37°C in a humidified incubator with 5% CO₂

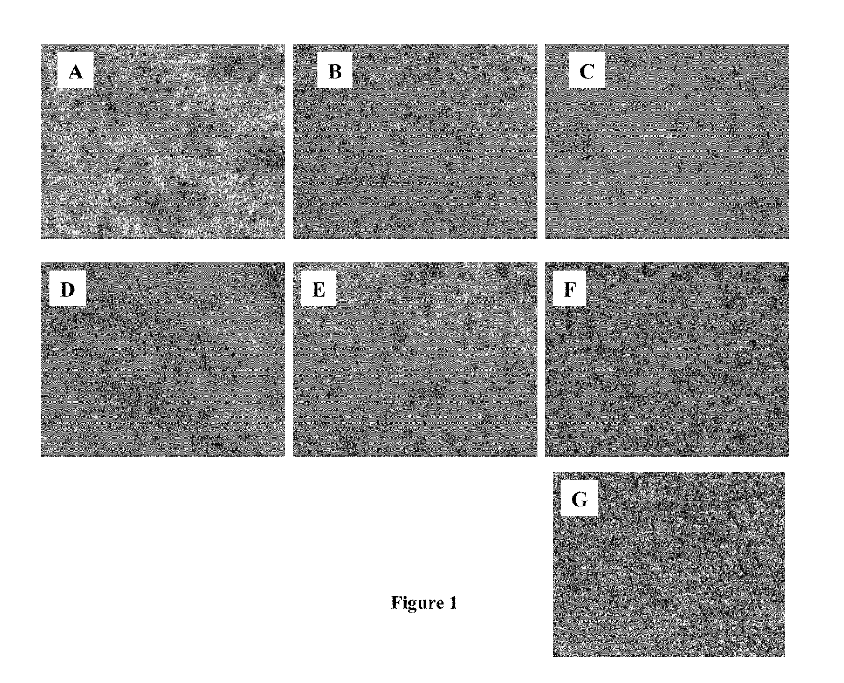

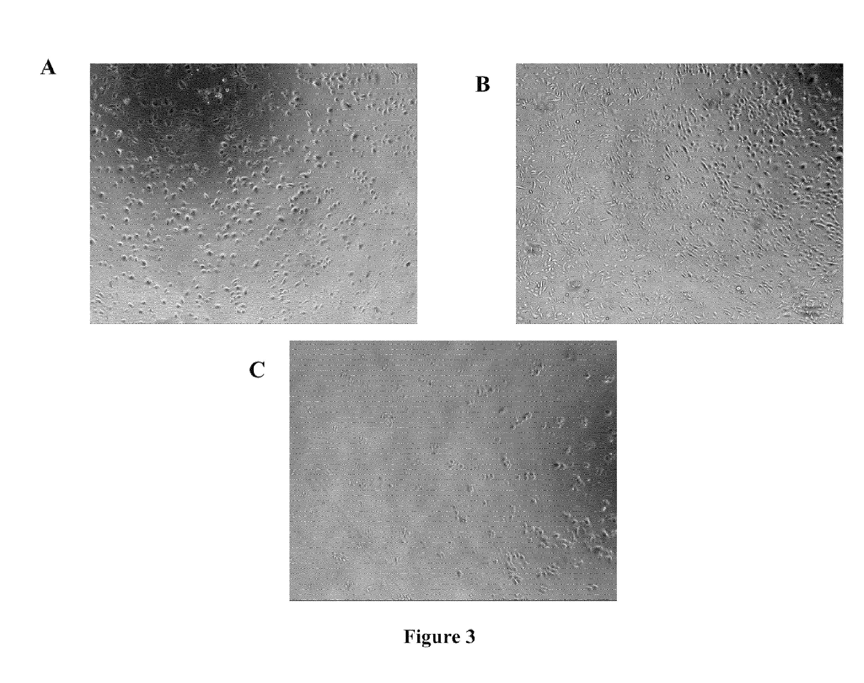

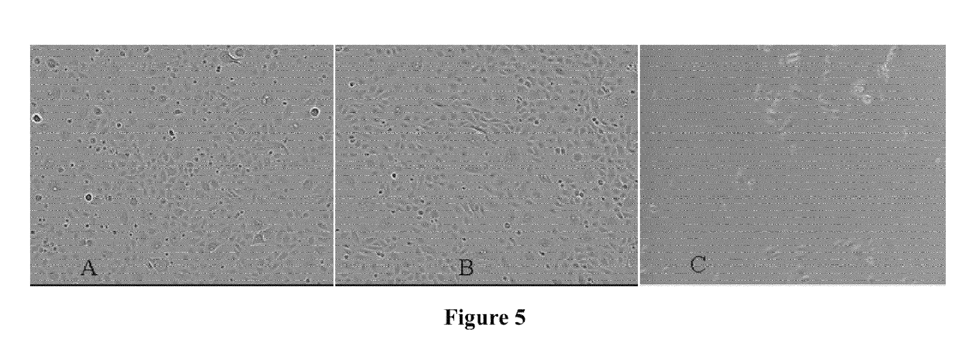

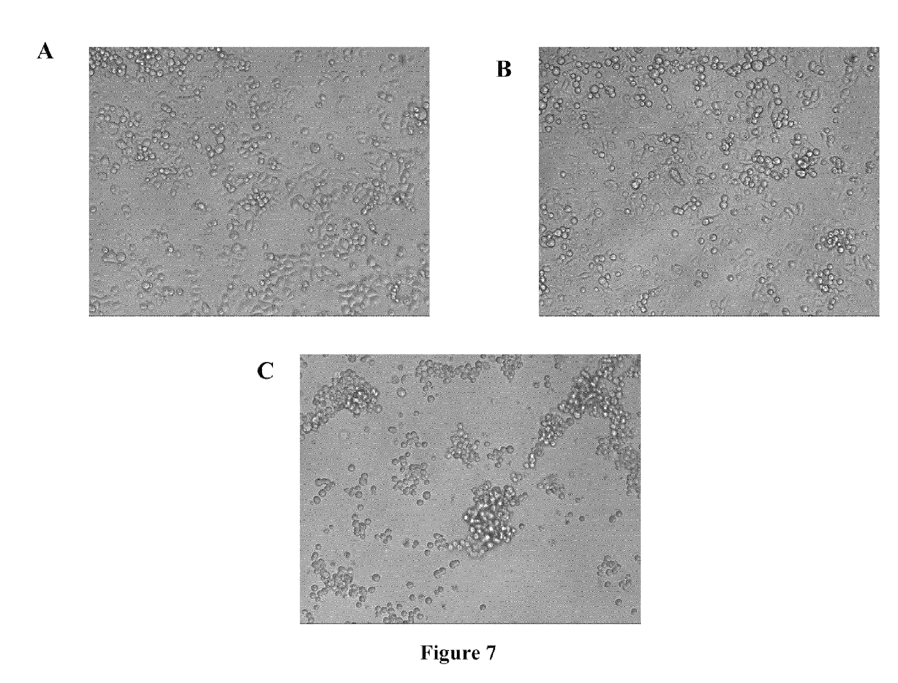

Following 24-48 hrs incubation post-seeding, cells were visualized with the aid of a microscope and images captured. Notably, cell attachment, spreading and proliferation was comparable between a surface with a coating thereon of a compound having amino acid sequence GPCGPPGPPGPPGPPGPPGFX₁GERGPPGPPGPPGPPGPPGPC (SEQ ID NO: 1) wherein X₁ represents hydroxyproline or collagen (*i.e.,* BD BioCoat Collagen1) whereas cell attachment, spreading and proliferation was significantly reduced in tissue culture treated surface without a coating thereon. This pattern was evident in human hepatocytes *(see* Figure 1A-G), human keratinocytes (see Figures 3 and 5) and human pancreatic cancer cells *(see* Figure 7).

In addition to the aforementioned visual analysis, MTS analysis was carried out day4-day5 post plating to quantify the number of cells. In brief, media was removed by aspiration and 300 µl complete media containing MTS (Promega cat # G3582) was added to each well of a 24 well plate. Cells were incubated for 1 hr at 37°C in a humidified incubator at 5% CO₂. Following incubation, 0.1 ml media was transferred to BD Falcon™ 96 well plate and absorbance was measured at 490 nm.

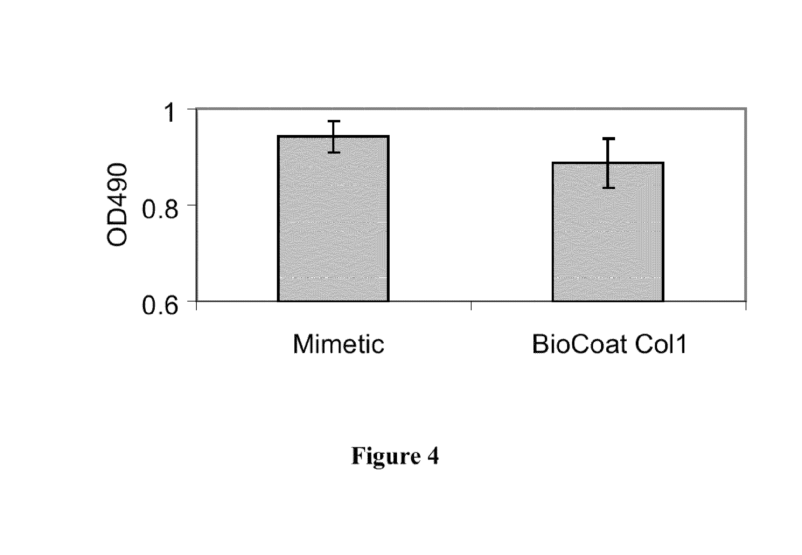

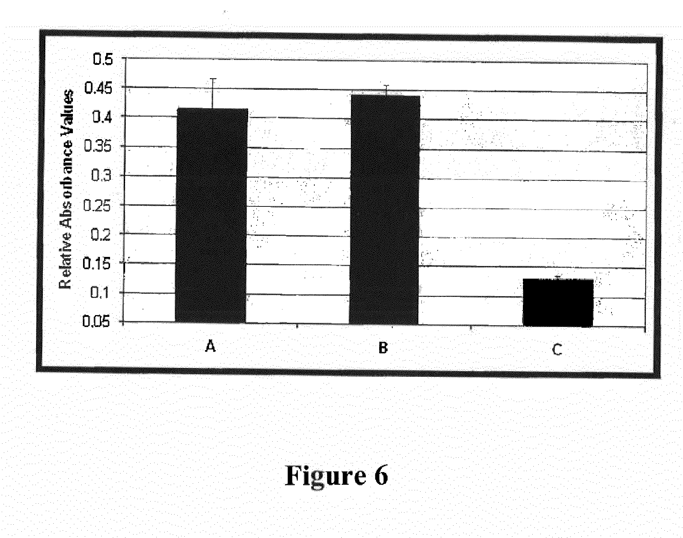

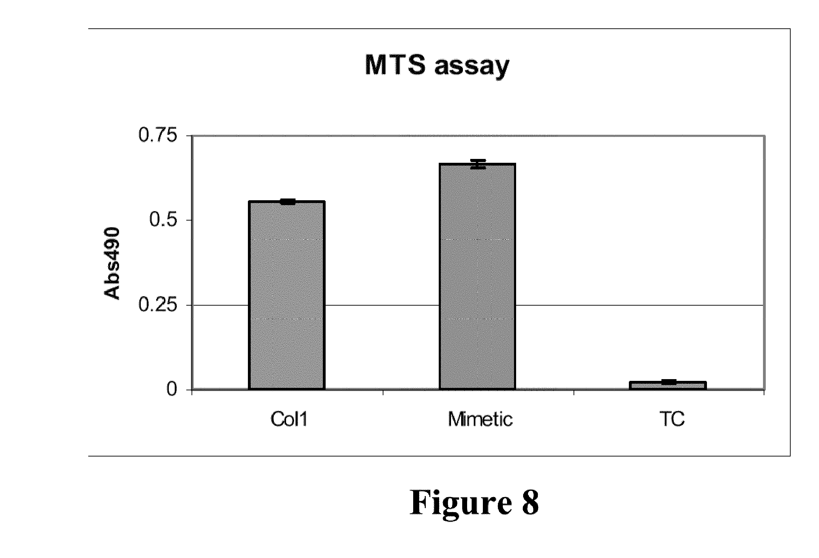

In accordance with the visual observations discussed above, surfaces with a coating thereon of GPCGPPGPPGPPGPPGPPGFX₁GERGPPGPPGPPGPPGPPGPC (SEQ ID NO: 1) wherein X₁ represents hydroxyproline or collagen (*i.e.,* BD BioCoat Collagen1) had a comparable number of cells whereas the number of cells in tissue culture surface without any coating was significantly reduced. This pattern was evident in both human keratinocytes (*see* Figures 4 and 6) and human pancreatic cancer cells *(see* Figure 8).

CYP3A4 induction was commenced 18 to 24hrs post-plating of human hepatocytes using rifampicin as an inducer following the supplier's (BD BioSciences) protocol including the recommended concentration for induction. Negative control included DMSO only. During induction cells were incubated in a humidified incubator at 37°C with 5% CO₂ for 24hr ± 6 hr. The induction step was repeated for 2 subsequent days, a total of 3 days.

After freshly plated hepatocytes were induced for ∼72 hr ± 8 hr, media was aspirated and cells were washed with pre-warmed Hepato-STIM media before conducting the CYP3A4 enzyme assay using testosterone as a substrate. A testosterone reaction mix was prepared following the supplier's (BD BioSciences) protocol. Following aspiration of the media, 400 µl testosterone reaction mix was added to each well of a 24-well plate. The cells were incubated for 30 min at 37°C in a humidified incubator with 5% CO₂. At the end of the incubation period, 300 µl was removed from each well, placed in an eppendorf tube and stored on ice. The reaction was stopped by adding 150 µl acetonitrile to each tube. The samples were then spun for 5 min at room temp at 14,000 rpm and the supernatant transferred to another tube. The supernatant was subsequently analyzed by HPLC. A standard curve was prepared using 6-β-testosterone from which the amount of 6-β-testosterone formed by the cells was quantified.

The remaining reaction mix was removed from the plates and the cells lysed in PBS + 1% SDS for the total protein from the cell lysate quantified using BCA kit (Pierce). Enzyme activity was reported as pmol product/min/ mg protein.

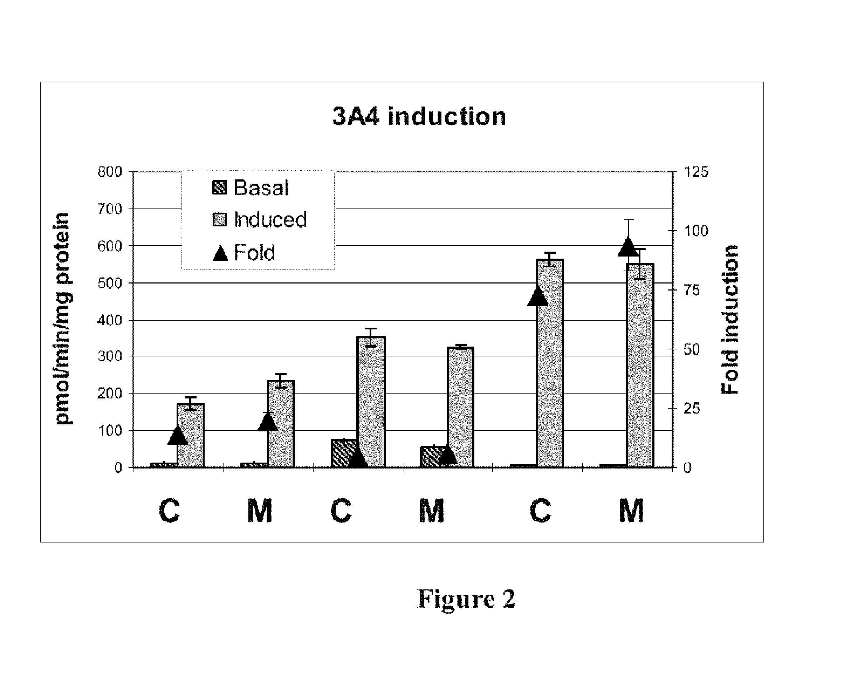

As illustrated in Figure 2, the level of basal activity of CYP3A4 as well as fold induction was comparable on surfaces with a coating thereon of GPCGPPGPPGPPGPPGPPGFX₁GERGPPGPPGPPGPPGPPGPC (SEQ ID NO: 1) wherein X₁ represents hydroxyproline or collagen (*i.e.,* BD BioCoat Collagen1).

It will be appreciated by those skilled in the art that changes could be made to the embodiments described above without departing from the broad inventive concept thereof It is understood, therefore, that this invention is not limited to the particular embodiments disclosed, but is intended to cover modifications that are within the spirit and scope of the invention, as defined by the appended claims.

## Claims

1. A method for cell culture comprising contacting a suspension of cells to a surface wherein at least a portion of the surface comprises a coating thereon of a compound comprising amino acid sequence GPCGPPGPPGPPGPPGPPGFX₁GERGPPGPPGPPGPPGPPGPC (SEQ ID NO: 1) wherein X₁ represents hydroxyproline and incubating the cells under conditions suitable for cell culture; wherein cell attachment to the surface is comparable to cell attachment of the suspension of cells to a collagen coated surface.

2. The method of Claim 1, wherein the cells are keratinocytes.

3. The method of Claim 1, wherein the cells are pancreatic cancer cells.

4. The method of Claim 1, wherein the cells are hepatocytes.

5. The method of Claim 1, wherein the number of cells attached to the surface is at least 2-fold greater relative to a control surface without any extracellular matrix protein coating thereon.

6. The method of Claim 2, wherein the number of cells attached to the surface is at least 3-fold greater relative to a control surface without any extracellular matrix protein coating thereon.

7. The method of Claim 3, wherein the number of cells attached to the surface is at least 10-fold greater relative to a control surface without any extracellular matrix protein coating thereon.

8. The method of Claim 4, wherein the level of basal activity of cytochrome P₄₅₀ 3A4 in hepatocytes attached to the surface is comparable to the level of basal activity of cytochrome P₄₅₀ 3A4 in hepatocytes attached to a collagen coated surface.

9. The method of Claim 4, wherein cytochrome P₄₅₀ 3A4 induction is comparable to the level of cytochrome P450 induction of hepatocytes attached to a collagen coated surface.

10. The method of Claim 1, wherein the surface is a cell culture vessel.

11. The method of Claim 1, wherein the surface is a microcarrier.

12. The method of Claim 1, wherein the cells are incubated at 37°C in a humidified incubator with 5% CO₂.

13. The method of Claim 1, wherein the cells are incubated for at least 24 hours.

14. A cell cultured using the method of Claim 1.


SEQUENCE LISTING

<110>  Becton, Dickinson and Company

<120>  Methods for Cell Culture Using A Synthetic, Defined Collagen
       Mimetic Surface

<130>  P-9198 (102-822)

<150>  US 13/310,988
<151>  2011-12-05

<150>  US 61/420,860
<151>  2010-12-08

<160>  1

<170>  PatentIn version 3.5

<210>  1
<211>  42
<212>  PRT
<213>  Artificial Sequence

<220>
<223>  Synthetic sequence


<220>
<221>  misc_feature
<222>  (21)..(21)
<223>  Xaa denotes hydroxyproline

<400>  1

Gly Pro Cys Gly Pro Pro Gly Pro Pro Gly Pro Pro Gly Pro Pro Gly
1               5                   10                  15


Pro Pro Gly Phe Xaa Gly Glu Arg Gly Pro Pro Gly Pro Pro Gly Pro
            20                  25                  30


Pro Gly Pro Pro Gly Pro Pro Gly Pro Cys
        35                  40